# EUROPEAN PATENT SPECIFICATION

(11) **EP 0 815 241 B1**
(45) Date of publication and mention of the grant of the patent: **18.06.2003**
(21) Application number: 96909772.4
(22) Date of filing: 19.03.1996
(51) Int. Cl.: C12N 15/55, C12N 15/81, C12N 9/22

(54) **IDENTIFICATION AND CLONING OF A MOBILE TRANSPOSON FROM ASPERGILLUS**
IDENTIFIKATION UND KLONIERUNG EINES MOBILEN ASPERGILLUS TRANSPOSONS
IDENTIFICATION ET CLONAGE D'UN TRANSPOSON MOBILE A PARTIR D'ASPERGILLUS

(30) Priority: 21.03.1995 US 408413
(43) Date of publication of application: 07.01.1998
(73) Proprietor: GENENCOR INTERNATIONAL, INC., Palo Alto, California 94304 (US)
(72) Inventor: AMUTAN, Maria, Palo Alto, CA 94304-1013 (US); DUNN-COLEMAN, Nigel, S., Palo Alto, CA 94304-1013 (US); NYYSSONEN, Eini, M., Palo Alto, CA 94304-1013 (US)
(74) Representative: Kiddle, Simon John
(86) International application number: US9603734
(87) International publication number: WO96029414

(56) References cited:
- MOL. GEN. GENET. (1992), 232(1), 12-16 CODEN: MGGEAE;ISSN: 0026-8925, 1992, XP002008351 DABOUSSI, MARIE JOSEE ET AL: "Fot1, a new family of fungal transposable elements" cited in the application
- GENETICS (1993), 134(3), 859-68 CODEN: GENTAE;ISSN: 0016-6731, 1993, XP000576390 LINDHOLM, DAN ANDERS ET AL: "The transposable element mariner mediates germline transformation in Drosophila melanogaster"
- GENETICS (1990), 125(1), 103-14 CODEN: GENTAE;ISSN: 0016-6731, 1990, XP000576439 BRYAN, GLENN ET AL: "Insertion and excision of the transposable element mariner in Drosophila"
- J. BACTERIOL. (1990), 172(7), 3830-6 CODEN: JOBAAY;ISSN: 0021-9193, 1990, XP000576437 TENZEN, TOYOAKI ET AL: "Site-specific transposition of insertion sequence IS630"

### SEQUENCE LISTING

(1) GENERAL INFORMATION:
   (i) APPLICANT: Amutan, Maria
      Dunn-Coleman, Nigel
      Nyyssonen, Eini M.
   (ii) TITLE OF INVENTION: Identification of and Cloning a Mobile Transposon from Aspergillus
   (iii) NUMBER OF SEQUENCES: 12
   (iv) CORRESPONDENCE ADDRESS:
      (A) NAME: Genencor International, Inc.
      (B) STREET: 180 Kimball Way
      (C) CITY: So. San Francisco
      (D) STATE: CA
      (E) COUNTRY: USA
      (F) POSTAL CODE (ZIP): 94080
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) CURRENT APPLICATION DATA:
      (A) APPLICATION NUMBER: US
      (B) FILING DATE: March 21, 1995
      (C) CLASSIFICATION:
   (viii) ATTORNEY/AGENT INFORMATION:
      (A) NAME: Horn, Margaret A.
      (B) REGISTRATION NUMBER: 33,401
      (C) REFERENCE/DOCKET NUMBER: GC270
   (xi) TELECOMMUNICATION INFORMATION:
      (A) TELEPHONE: (415) 742-7536
      (B) TELEFAX: (415) 742-7217
(2) INFORMATION FOR SEQ ID NO: 1:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 1:
   GAGGAAAGCC AAGTA 15
(2) INFORMATION FOR SEQ ID NO: 2:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 2:
   TATGTTCTGC ATGGAG 16
(2) INFORMATION FOR SEQ ID NO: 3:
   (i) SEQUENCE CHARACTERISTICS:
      (A) LENGTH: 437 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 3:
   ACGTAATCAA CGGTCGAACG GGCCACACGG TCAGGCGGGC CATCCTGAAA TCCCATATAA 60
   AAGATGTCTT GGGGATTCTA TTATATATCA ACCAGTACTA CTTCTATGAA GCTCTAACTT 120
   TGTAGATAGT TATATATATA AGAATAAGTA TTCCATGAAT TTTTCAGATT TTAGAATTTT 180
   TACTTTGATA ATGAAACCAG ATTCTTATAT AAAACATATA AATACAGATA TTGTAATATG 240
   ATAAGTCCAT AAGTAAAAGT ATATTCATTT TTAGAAGGTA TATAGATATT ATTTATATTA 300
   TTTAAAATCT ATATAGAAGA AATCTAATTC TTCTAGACCT GGATGGTAGA GATATATTAT 360
   GTTTAAAAAG ATATCTTTTG TATAGTATTA CCAGATGGCC CGCCTGACCG TGTGGCCCGT 420
   CCGACCGTTG ATTACGT 437
(2) INFORMATION FOR SEQ ID NO: 4:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 4:
   ACGTAATCAA CGGTCGAACG GGCCACACGG TCAGGCGGGC CATC 44
(2) INFORMATION FOR SEQ ID NO: 5:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 5:
   GATGGCCCGC CTGACCGTGT GGCCCGTCCG ACCGTTGATT ACGT 44
(2) INFORMATION FOR SEQ ID NO: 6:
   (i) SEQUENCE CHARACTERISTICS:
      (A) LENGTH: 2315 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 6:
   ACGTAATCAA CGGTCGGACG GGCCACACGG TCAGGCGGGC CATCCCTTCG AAAACACCAC    60
   CTTGAATCAC CTACCCGAGG CTTTTCAACC ACCACAAATG CCACCAAAAG CATCTATCCC   120
   ATCAAAATCG CAGGTGGAGC AGGAAGGCAG GATTCTTCTT GCCATTGAAG CTATTCAGAA   180
   AGGCCAAATC ACTAGTATTC GTGAAGCAGC GCGTGTTTAT GACGTCGCTC GAACTACTCT   240
   CCAGGCTCGA TTATCTGGAC GTGTTTTCGC TAAAAATATG ACCAACGCAC GTCAAAAATT   300
   GTCAAATAAT GAAGAGGAAT CGCTTGTTAA ATGGATCCTA TCTCTAGATA AGCGAGGAGC   360
   AAGCCCCCGG CCACTTGATA TCAGAGATAT GGCTAATTTG ATTATCTCTA AACGAGGTTA   420
   TTCAACTGTT GAACAAGTAG GCATCAACTG GGCTTATAGC TTTGTTAAAC GCCACGAATC   480
   CCTACGAACT CGATTTGCTA GACGACTCAA CTATCCAAGA GCTAAAATGG AGGATCCTGA   540
   AGTTATAAAA GACTGGTTCC AACGCGTACA GGAAGTTATT CAAGAGTACG GGATCTCATC   600
   AGATGATATA TACAATTTCG ATGAAACAGG GTTTGCTATG GGAATGATTG CTACATATAA   660
   AGTAGTAACT AGTTCCCAGA GGGCAGGTCG GCCGTCCCTA GTTCAACCAG GGAATCGGGA   720
   ATGGGTCACT CCAATTGAGT GTATTCGCTC TAATGGAGAG GTTCTACCTT CGACCCTGAT   780
   CTTTAAAGGC AAAACACATC TAAAGGCATG GTATGAAGGT CAATCTATTC CTCCTACCTG   840
   GAGATTTGAA GTCAGTGATA ATGGTTGGAC TACTGATAAA ATTGGACTTC GATGGCTTCC   900
   AAAACACTTC ATTCCCTTGA TTAGAGGCAA ATCAGTAGGC AAATATAGCC TCCTAGTCCT   960
   CGATGGCCAC GGTAGTCATT TGACACCTGA ATTCGACCAA TCCTGTGCTG AAAATGAGGT  1020
   TATACCTATT TGTATGCCAG CTCATTCGTC CCATCTACTT CAGCCTCTTG ATGTTGGTTG  1080
   TTTTAGTGTG CTTAAACGCA CGTACGGAGG CATGGTTCCC AAGCAGATGC AATACGGCCG  1140
   CAATCATATC GACAAGCTTG ACTTCTTAGA GGTCTATCCT AAAGCTCACC AGTGTGCTTT  1200
   ATCAAAGTCG AATATAATCA GTGGTTTTAG AGCAACAGGT CTTGTTCCTC TAGATCCTGA  1260
   TCAAGTGCTT TCTCGACTCC ATATTCGCTT GAAAACACCA CCAACCCCGG ATAGCCAGTC  1320
   AAGTGGCTCA GTGCTTCAAA CACCACATAA TATAAAACAC CTTTTGGAGC ATCCAAAATC  1380
   AGTGGAACGC CTACTTCGGA AACGGCAAGC AAGTCCAACT TCACCTACAA ACTCTACACT  1440
   ACGTCAGCTT CTCAAAGGGT GTGAACTAGC AATAACAAAC TCAATCATAC TGGCTAAGGA  1500
   GAATGCGGAA TTACGTGCTA GCCATGAAAA GCAACTACCA AAGAGGAAGC GTTCAAGGAA  1560
   GCAGGTGATC TATACAGAAG GCACTACCGT TGAAGAGGCC CAGAGAGCTA TACAGGAAGT    1620
   GGAAGAGGTG CAGAATGATG AAGATATTGA GGTTGAACCC CAATCTCAAT ATACGGAGAC    1680
   CCCCTCGCGC GCGCCTCCAC GCTGCAGTAA TTGCTTCAAT ATAGGCCACC GACGTACACA    1740
   GTGTTCTAAA CCACCTACTA ATTAGTTAGA TAGCTGTTTT TACAAGCATT TATGTTGATT    1800
   TAGAGGCCTC ATTTGGATCA TATCGGGTAA TCCTACCGGG AGATGGCCCG CCTGACCGTG    1860
   TGGCCCGCCC GACCGTTGAT TACGTNNNNN ACGTAATCAA CGGTCGGACG GGCCCCCCGG    1920
   TCCGGCGGGC CATCTGGTAA TACTATACCA AAGATATCTT TTTAAACATA ATATATCTCT    1980
   ACCATCCAGG TCTAGGAGAA TTAGATTTCT TCTATATAGA TTTTAAATAA TATAAATAAT    2040
   ATCTATATAC CTTCTAAAAA TGAATATACT TTTACTTATG GACTTATCAT CTGTATTTAT    2100
   ATGTATTATA TAAGAATCTG GTTTCATTAT CAAAGTAAAA ATTCTAAAAT CTGAAAAATT    2160
   CATGGAATAC TTATTCTTAT ATATATAACT ATCTACAAAG TTAGAGCTTC ATAGAAGTAG    2220
   TACTGGTTGA TATATAATAG AATCCCCAAG ACATCTTTTA TATGGGATTT CAGGATGGCC    2280
   GCCGACCGTG TGGCCCGTCC GACCGTTGAT TACGT                               2315
(2) INFORMATION FOR SEQ ID NO: 7:
   (i) SEQUENCE CHARACTERISTICS:
      (A) LENGTH: 555 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 7:
   Met Pro Pro Lys Ala Ser Ile Pro Ser Lys Ser Gln Val Glu Gln Glu
   1 5 10 15
   Gly Arg Ile Leu Leu Ala Ile Glu Ala Ile Gln Lys Gly Gln Ile Thr
   20 25 30
   Ser Ile Arg Glu Ala Ala Arg Val Tyr Asp Val Ala Arg Thr Thr Leu
   35 40 45
   Gln Ala Arg Leu Ser Gly Arg Val Phe Ala Lys Asn Met Thr Asn Ala
   50 55 60
   Arg Gln Lys Leu Ser Asn Asn Glu Glu Glu Ser Leu Val Lys Trp Ile
   65 70 75 80
   Leu Ser Leu Asp Lys Arg Gly Ala Ser Pro Arg Pro Leu Asp Ile Arg
   85 90 95
   Asp Met Ala Asn Leu Ile Ile Ser Lys Arg Gly Tyr Ser Thr Val Glu
   100 105 110
   Gln Val Gly Ile Asn Trp Ala Tyr Ser Phe Val Lys Arg His Glu Ser
   115 120 125
   Leu Arg Thr Arg Phe Ala Arg Arg Leu Asn Tyr Pro Arg Ala Lys Met
   130 135 140
   Glu Asp Pro Glu Val Ile Lys Asp Trp Phe Gln Arg Val Gln Glu Val
   145                 150                 155                 160
   Ile Gln Glu Tyr Gly Ile Ser Ser Asp Asp Ile Tyr Asn Phe Asp Glu
                   165                 170                 175
   Thr Gly Phe Ala Met Gly Met Ile Ala Thr Tyr Lys Val Val Thr Ser
               180                 185                 190
   Ser Gln Arg Ala Gly Arg Pro Ser Leu Val Gln Pro Gly Asn Arg Glu
           195                 200                 205
   Trp Val Thr Pro Ile Glu Cys Ile Arg Ser Asn Gly Glu Val Leu Pro
       210                 215                 220
   Ser Thr Leu Ile Phe Lys Gly Lys Thr His Leu Lys Ala Trp Tyr Glu
   225                 230                 235                 240
   Gly Gln Ser Ile Pro Pro Thr Trp Arg Phe Glu Val Ser Asp Asn Gly
                   245                 250                 255
   Trp Thr Thr Asp Lys Ile Gly Leu Arg Trp Leu Pro Lys His Phe Ile
               260                 265                 270
   Pro Leu Ile Arg Gly Lys Ser Val Gly Lys Tyr Ser Leu Leu Val Leu
           275                 280                 285
   Asp Gly His Gly Ser His Leu Thr Pro Glu Phe Asp Gln Ser Cys Ala
       290                 295                 300
   Glu Asn Glu Val Ile Pro Ile Cys Met Pro Ala His Ser Ser His Leu
   305                 310                 315                 320
   Leu Gln Pro Leu Asp Val Gly Cys Phe Ser Val Leu Lys Arg Thr Tyr
                   325                 330                 335
   Gly Gly Met Val Pro Lys Gln Met Gln Tyr Gly Arg Asn His Ile Asp
               340                 345                 350
   Lys Leu Asp Phe Leu Glu Val Tyr Pro Lys Ala His Gln Cys Ala Leu
           355                 360                 365
   Ser Lys Ser Asn Ile Ile Ser Gly Phe Arg Ala Thr Gly Leu Val Pro
       370                 375                 380
   Leu Asp Pro Asp Gln Val Leu Ser Arg Leu His Ile Arg Leu Lys Thr
   385                 390                 395                 400
   Pro Pro Thr Pro Asp Ser Gln Ser Ser Gly Ser Val Leu Gln Thr Pro
                   405                 410                 415
   His Asn Ile Lys His Leu Leu Glu His Pro Lys Ser Val Glu Arg Leu
               420                 425                 430
   Leu Arg Lys Arg Gln Ala Ser Pro Thr Ser Pro Thr Asn Ser Thr Leu
           435                 440                 445
   Arg Gln Leu Leu Lys Gly Cys Glu Leu Ala Ile Thr Asn Ser Ile Ile
       450                 455                 460
   Leu Ala Lys Glu Asn Ala Glu Leu Arg Ala Ser His Glu Lys Gln Leu
   465                 470                 475                 480
   Pro Lys Arg Lys Arg Ser Arg Lys Gln Val Ile Tyr Thr Glu Gly Thr
                   485                 490                 495
   Thr Val Glu Glu Ala Gln Arg Ala Ile Gln Glu Val Glu Glu Val Gln
               500                 505                 510
   Asn Asp Glu Asp Ile Glu Val Glu Pro Gln Ser Gln Tyr Thr Glu Thr
           515                 520                 525
   Pro Ser Arg Ala Pro Pro Arg Cys Ser Asn Cys Phe Asn Ile Gly His
       530                 535                 540
   Arg Arg Thr Gln Cys Ser Lys Pro Pro Thr Asn
   545                 550                 555
(2) INFORMATION FOR SEQ ID NO: 8:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 8:
   CCAACCGAGT CCTCAGTATA GAC                                              23
(2) INFORMATION FOR SEQ ID NO: 9:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 9:
   CAACGCTTCA TAGGCGTCCA GATC                                             24
(2) INFORMATION FOR SEQ ID NO: 10:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 10:
   ATATGAATTC ACGTAATCAA CGGTCGGACG GGCCACACGG TCAGGCGGGC CATC 54
(2) INFORMATION FOR SEQ ID NO: 11:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 11:
   ATATGAATTC CTTCTTGACT TCCCCGGAAC 30
(2) INFORMATION FOR SEQ ID NO: 12:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 12:
   ATATAAGCTT GTCACTGGAC GACATTTCAG 30

## Description

### FIELD OF THE INVENTION

The present invention is directed at the identification, cloning and sequencing of a mobile transposon or transposable element from *Aspergillus niger* var. *awamori*. The transposable element, referred to as Vader, is an approximately 440 base pair (bp) element bounded by an inverted repeat sequence of approximately 44 base pairs. The transposable element targets a "TA" sequence in target DNA during insertion. In addition, the present invention is directed at the identification, cloning and sequencing of one or more transposable element(s) from other filamentous fungi using as a probe DNA comprising the 44 bp inverted repeat isolated from *Aspergillus niger* var. *awamori*. Also provided are methods for making mutants using the transposable element(s) of the present invention by inactivating genes via insertion of the transposable element(s) into a target gene and, alternatively, to activate or turn on genes using the transposable element(s) as described herein.

### BACKGROUND OF THE INVENTION

It is well know that DNA segments, called transposons, are able to insert into many sites in the genome of their host organisms. Transposons are known in both prokaryotes, such as bacteria, as well as in eukaryotes, although there have been few transposons isolated from filamentous fungi.

Several groups have looked for transposons in filamentous fungi. The element *pogo,* which exists in multiple copies and at different sites in different strains of *Neurospora crassa,* was described by Schectman (1) and is believed to be a transposon. To date the most characterized transposon in filamentous fungi is Tad. Tad was isolated as a spontaneous mutant in the *am* (glutamate dehydrogenase) gene in an Adiopodoume strain of *N. crassa* isolated from the Ivory Coast. To detect mutations caused by insertion of a transposable element, Kinsey and Helber (2) isolated genomic DNA from 33 *am* mutant strains which were then screened by Southern analysis for restriction fragment size alterations. In two of the mutant strains, the mutation was shown to be caused by the insertion of a 7 kb element (Tad) into the *am* gene. Subsequently Kinsey (3) demonstrated that Tad was able to transpose between nuclei of heterokaryons, confirming that Tad was a retrotransposon and that there was a cytoplasmic phase involved in the retrotransposition events. More recently, Cambareri *et al*. (4) demonstrated that Tad was a LINE-like DNA element with two major open reading frames (ORFs) on the plus strand. Typical of LINE-like elements, Tad had no terminal repeats. Attempts to isolate mobile transposons in laboratory strains of *N. crassa* were unsuccessful.

A second retrotransposon was cloned by McHale *et al*. (5), who reported the isolation of CfT-1, an LTR-retrotransposon from *Cladosporium fulvum*. This transposon was 6968 bp in length and bounded by identical long terminal repeats of 427 bp, a 5 bp target site duplication. Virus-like particles were detected which co-sediment with reverse transcriptase activity in homogenates of this fungus.

Daboussi et *al* (6) were the first to successfully use the *niaD* (nitrate reductase) gene as a transposon trap. The *niaD* mutants can be isolated by a direct selection for chlorate resistance (7). The strategy employed was to isolate *niaD* mutants amongst six isolates belonging to different races of the fungus *Fusarium oxysporum*. More than 100 *niaD* mutants were isolated from each isolate and examined for instability. One strain, F24, yielded up to 10% unstable *niaD* mutants. Assuming that the genetic instability of the *niaD* mutants was caused by transposable elements, it seemed plausible that this isolate contained mobile transposons. A stable *niaD* mutant in the F24 was transformed with the cloned *niaD* gene from *A. nidulans* because the *F. oxysporum niaD* gene had not been cloned. Unstable *niaD* mutants were isolated in transformants containing the *A. nidulans niaD* gene. Two unstable *niaD* mutants were shown by Southern blot analysis to contain a insertion of 1.9 kb in size. Analysis of this element, *Fot1*, revealed it was 1928 bp long, had a 44 bp inverted terminal repeats, contained a large open reading frame, and was flanked by a 2 bp (TA) target site duplication. Very recently, Daboussi *et al.* (8) have reported the cloning of a new transposable element from an unstable *niaD* mutant . This element, *FML* (*Fusarium mariner*-like), is 1280 bp long and has inverted repeats of 27 bp. The *FML* element inserts into a TA site and excises imprecisely.

Using the characterization of unstable *niaD* mutants strategy, Lebrun et *al.* (9) were able to isolate a transposon from *Magnaporthe grisea*. However, in this case the *A. nidulans niaD* gene which was transformed into *M. grisea* by transformation was used as a transposon trap. The element inserted into the *niaD* gene was shown to belong to a family of *M. grisea* LTR-retrotransposons, Fos1 (Schull and Hamer, unpublished) and Mag1 (Farman and Leong, unpublished). The cloned retro-element was 5.6 kb and the target site (ATATT) was shown to be duplicated. All revertants from this mutant examined had one copy of the LTR left at the point of insertion. A second transposon, Pot2, from *M. grisea* was recently cloned by Kachroo *et al*. (10). The strategy used to clone Pot2 was to analyze the fingerprint patterns of repetitive DNA's which were cloned from the *M. grisea* genome. A repetitive family present in both rice and non-rice pathogens of *M. grisea* in high copy number was cloned. The element, 1857 bp in size, has a 43 bp perfect terminal inverted repeats (TIR) and 16 bp direct repeats within the TIRs. An open reading frame was shown to display extensive identity to that of Fot1 of *F. oxysporum*. As with Fot1, the Pot2 element duplicates the dinucleotide TA at the target insertion site. Pot2 was shown to be present at a copy number of approximately 100 per haploid genome.

Several groups have reported looking without success for transposons in laboratory strains of *A. nidulans* (Kinghorn personnel communication, 5). One explanation for the lack of transposons in laboratory strains is that the desirable features of strain stability required for genetic analysis may preclude strains with mobile transposon. By using the *niaD* gene as a transposon trap we have identified and isolated a transposable element from the industrially important fungus *A. niger var. awamori*. This element, Vader, is present in approximately 15 copies in *A. niger and A. nigel* var. *awamori*. Southern analysis of *A. nidulans* with this element indicates that this transposable element was absent from one laboratory strain and only present as a single copy in a second laboratory strain. These results support the notion that laboratory strains of *A. nidulans* contain very few transposons.

### BRIEF DESCRIPTION OF THE INVENTION

In accordance with the present invention, a novel eukaryotic transposable element from *Aspergillus niger* var. *awamori* is provided. The novel transposable element is an approximately 440 bp element which comprises a 44 bp inverted repeat sequence at either end of the transposable element. The target for insertion of this novel transposable element is a "TA" sequence in the target DNA for insertion. The "TA" sequence is repeated at either end of the transposon upon insertion of the transposable element into the target DNA.

Another embodiment of the present invention comprises a fragment of the transposable element comprising a 44 bp inverted repeat sequence found at either terminus of the transposable element from *A. niger* var. *awamori*, as well as the use of said 44 bp fragment as a DNA probe useful in isolating and/or cloning transposable elements from other filamentous fungi. Using the inverted repeat of Vader, a transposon from *A. niger var. awamori* (Tan) was cloned.

In a process embodiment of the present invention there are provided methods for gene tagging comprising using the transposable elements of the present invention to inactivate genes via insertion of the element into a given gene, thus disrupting or inactivating gene expression. Alternatively, the transposable element can be used in activation tagging (to activate or turn on genes) rather than for gene disruption. For example, by inserting DNA coding a promoter into the transposable element and then allowing such transposable element to become inserted 5' to a desired gene, the promoter may be activated to drive the expression of the desired gene product.

### BRIEF DESCRIPTION OF THE DRAWINGS

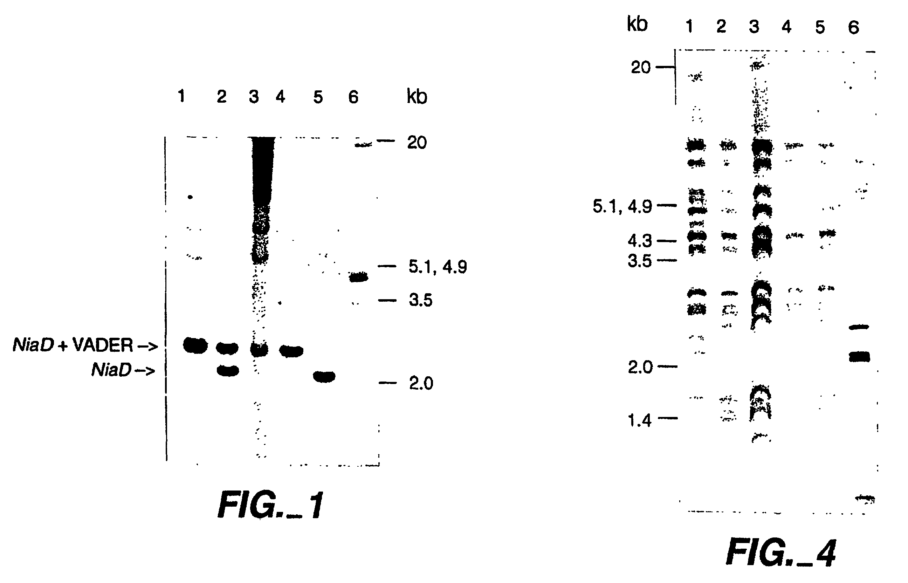

Fig. 1 shows the Southern blot analysis of unstable *niaD* mutants. Fungal genomic DNA from four *niaD* mutants and UVK143 were digested with BgIII (sites are 3' of all inserts). Blot probed with 500 bp fragment of Sall digested PCR product of niaD1 and niaD2. DIG-labeled probe concentration was 20 mg/mL prehybridization solution. Wild-type band hybridizes at 2.5 kb while gene with insertion hybridizes at 2.9 kb. Lanes: 1=*niaD*392; 2=*niaD* 587; 3=*niaD*436; 4=*niaD*410; 5=UVK143; 6=MW marker III (Boehringer Mannheim).

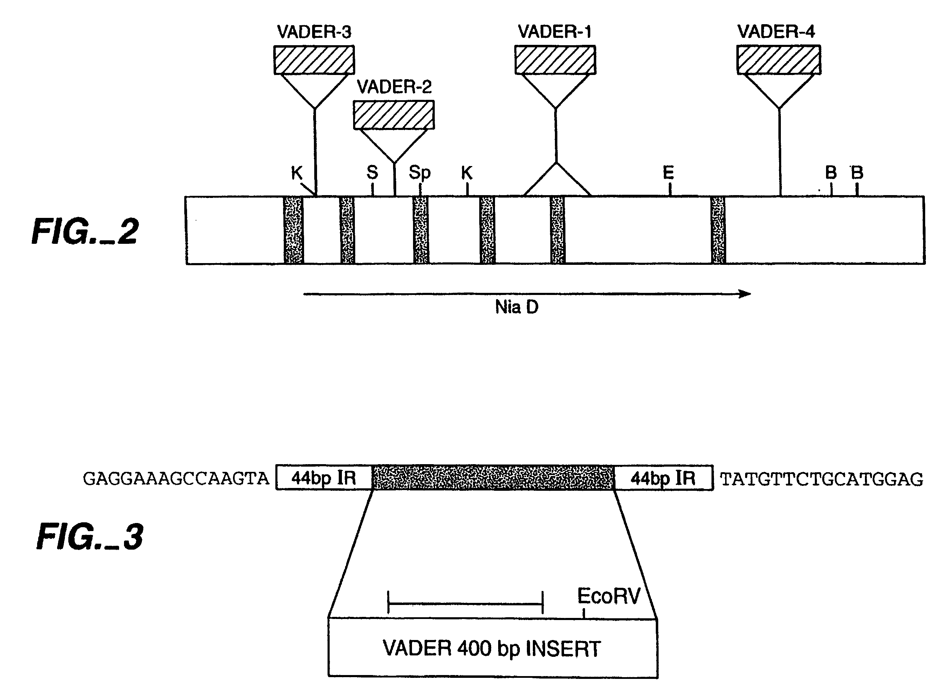

Fig. 2 depicts the mapping of Vader insertions within the *niaD* gene. The positions of Vader insertions 1-4 are shown relative to the six introns of the structural gene coding region. Because the exact site of insertion for Vader-4 is still unknown, it has been presented using the approximate area of its insertion. Relevant restriction sites are shown using the following letters: E=EcoRl, S=Sall, Sp=Sphl, K=Kpnl, and B=BgIII.

Fig. 3 depicts the location of Vader 2 in the *niaD* gene. The sequence shown here begins at +0.730 kb of *niaD.* The 2 bp TA duplication is indicated in bold and the 44 bp inverted repeats of Vader are boxed. The flanking region from the DNA sequence of the *niaD* gene are shown on either side of the Vader insert. Seq. ID No. 1 comprising flanking DNA from the *niaD* gene, is shown on the left (5') of the Vader insert, ending with the target sequence "TA." Seq. ID No. 2 comprising flanking DNA from the *niaD* gene, is shown on the right (3') of the Vader insert, starting with the target sequence "TA" duplication seen with the Vader insert.

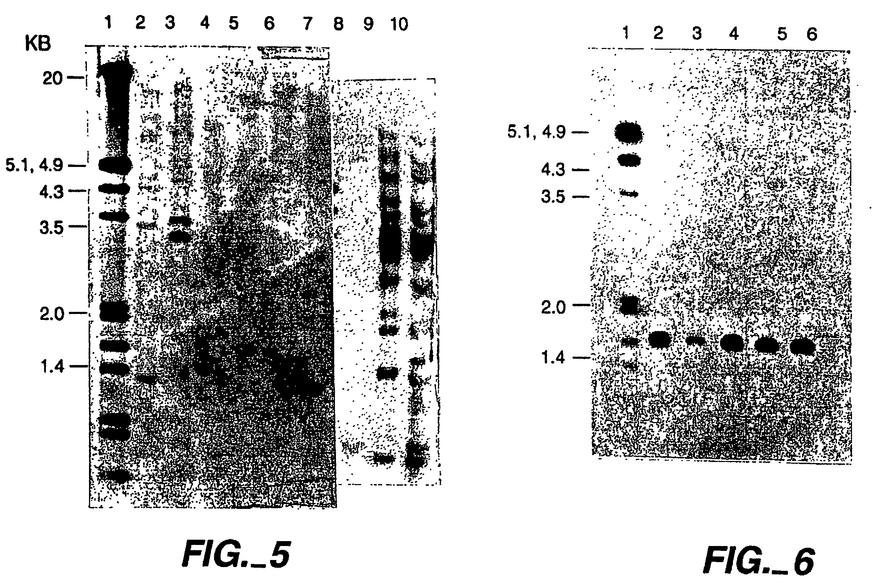

Fig. 4 shows Southern blot analysis to determine Vader genomic copy number. Four *A. niger* var. *awamori niaD* mutants and UVK143 were digested with EcoRV to completion. EcoRV cuts the Vader sequence once. Hybridization indicates that Vader is present in the genome in more than 14 copies. The hybridizing bands of *niaD* 392, which are different from the other mutants and UVK143, suggest that the sequence is mobile. Lanes: 1=MW marker III, 2=UVK143, 3=*niaD*410, 4=*niaD* 436, *5=niaD* 587, 6=*niaD* 392.

Fig. 5. Southern blot to determine presence of Vader sequence in other fungi. Other filamentous fungi, an industrial production strain and *niaD* mutant 392, were digested with EcoRV to completion. Low stringency hybridization indicates that sequences homologous to Vader are present in *A. nidulans* (FGSC A691), *A. cinnamomeus, A. phoenicis, A. foetidus*, an industrial *A. niger* strain. Lanes: 1=MW marker III, 2=*A. nidulans* (FGSC A691), 3= *A. cinnamomeus* (ATCC# 1027), 4=*A. versicolor*, 5=*A*. *wentii* (ATCC# 10593), 6=*A. nidulans* (FGSC A237), 7=*A. phoenicis* (ATCC# 11362), 8=A. *foetidus*, 9=an industrial glucoamylase production strain of *A. niger* (ETC# 2663), 10=*A. niger* var. *awamori niaD* mutant 392.

Fig. 6. Southern blot to determine Tan (transposon from *A. niger*) genomic copy number. Four *niaD* mutants *A. niger* var. *awamori* mutants and UVK143 were digested with EcoR1 to completion. EcoR1 cuts the Tan sequence once. Hybridization indicates that Tan is present as a single copy in the genome. Lanes: 1=MW marker III, 2=UVK143, 3=*niaD*410, 4=*niaD* 436, 5=*niaD* 587, 6=*niaD* 392.

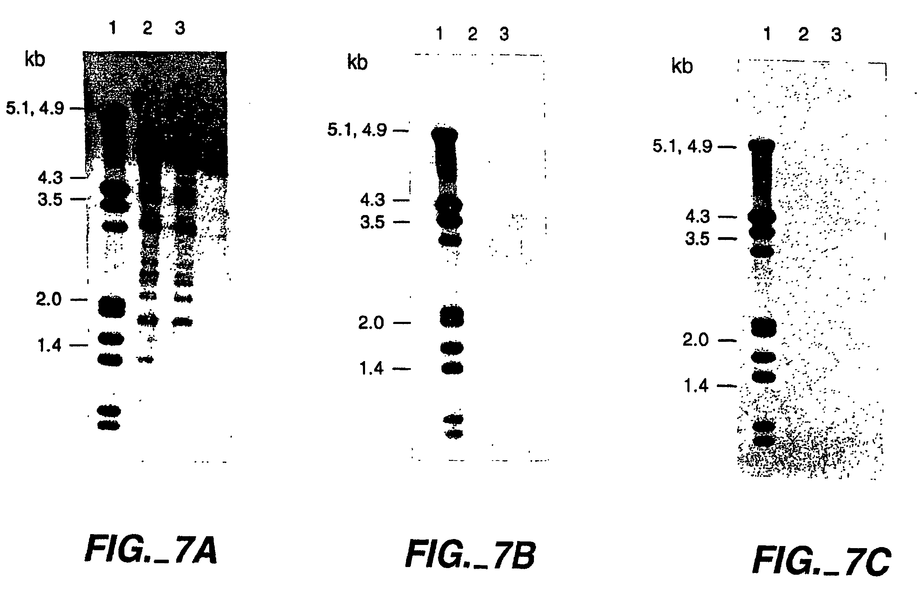

Fig. 7. Southern blots to determine if the inverted repeats of transposable elements Fot1 and Pot2 will hybridize to elements in *A. niger* var. *awamori*. Four *niaD* mutants *A. niger* var. *awamori* mutants were digested with EcoR1 to completion. EcoR1 cuts the Tan sequence once. Inverted repeat probes of Tan (Seq. ID No. 5), Fot1, Pot2 were oligo-tailed using Digoxigenin (Boehringer Mannheim, 1992). Lanes: 1=MW marker III. 2=*niaD*436, 3=*niaD*587. Blots A, B, and C were probed with the labeled inverted repeat probes of Tan, Fot1 and Pot2, respectively.

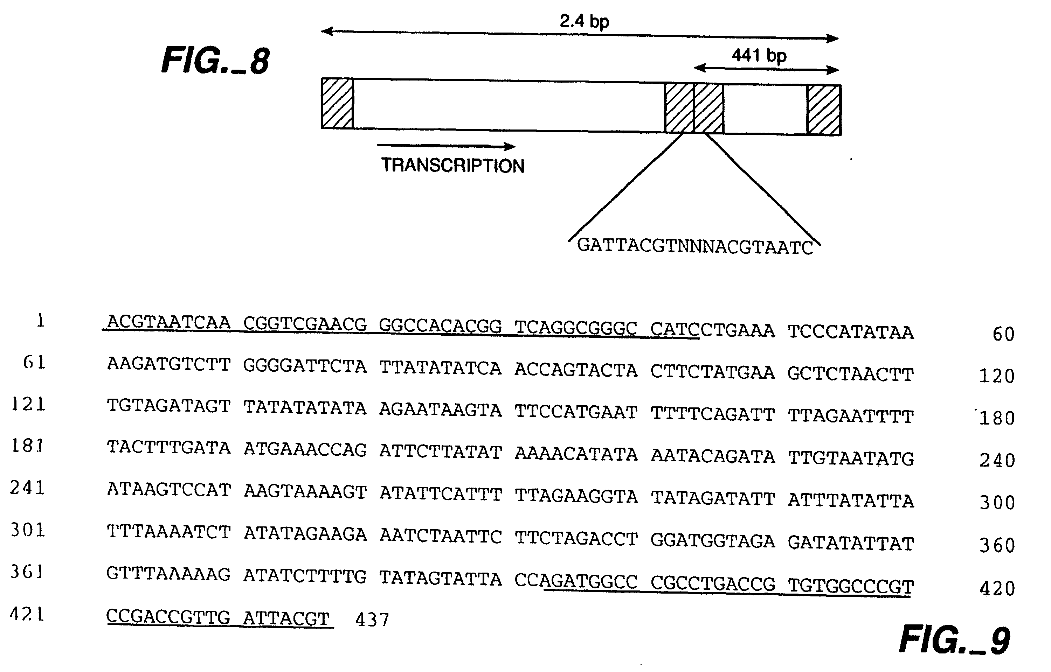

Fig. 8 diagramatically shows the Tan organization where the 2.4 kb element includes 4 inverted repeat sequences, as well as the approximately 440 bp Vader insertion sequence. Due to putative stem loop structure, the complete sequence of Tan in the dashed area between the larger fragment and Vader is unknown (as designated by "N" in the figure and corresponding sequences).

Fig. 9 shows the sequence of the Vader insertion (Seq. ID No. 3). Vader was found to be 437 bp in length. The 44 bp inverted repeat of the Vader insert corresponding to Seq. ID No. 4 and Seq. ID No. 5, respectively, from the 5' end to the 3' end of Vader are underlined, the single mismatch which occurs in the inverted repeats is identified in bold, and the TA 2 bp duplication is shown in bold print. *niaD* sequences flanking the element are shown in lower case letters.

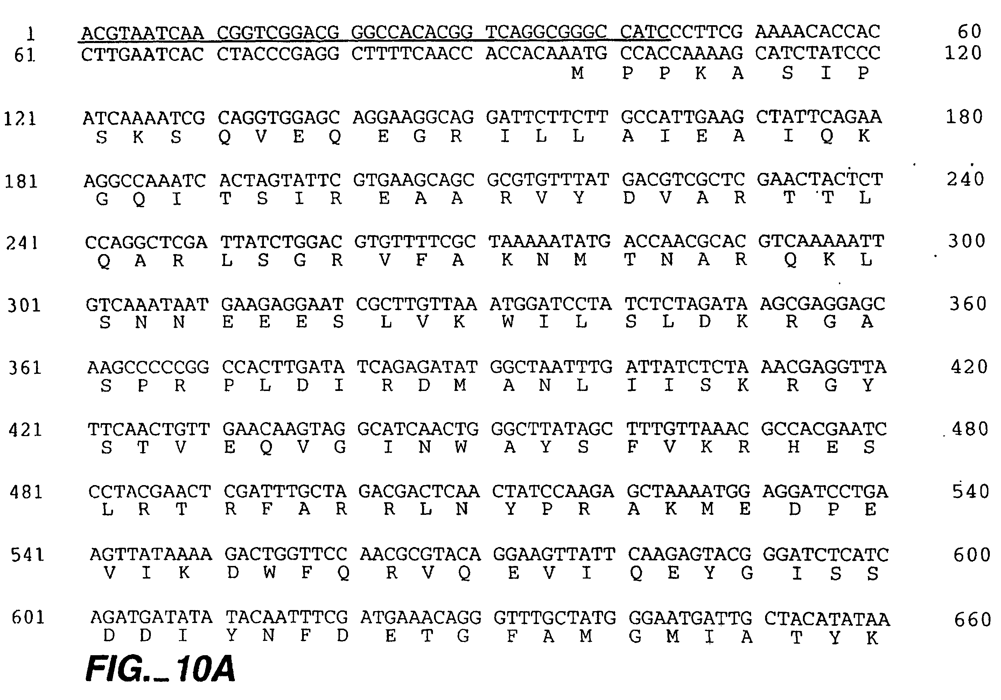

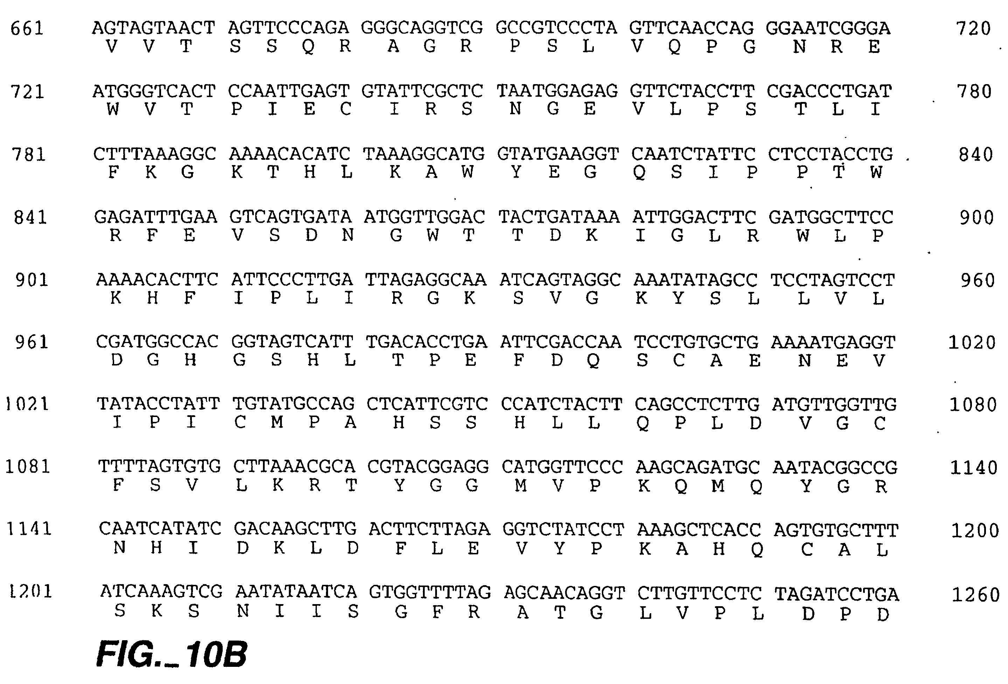

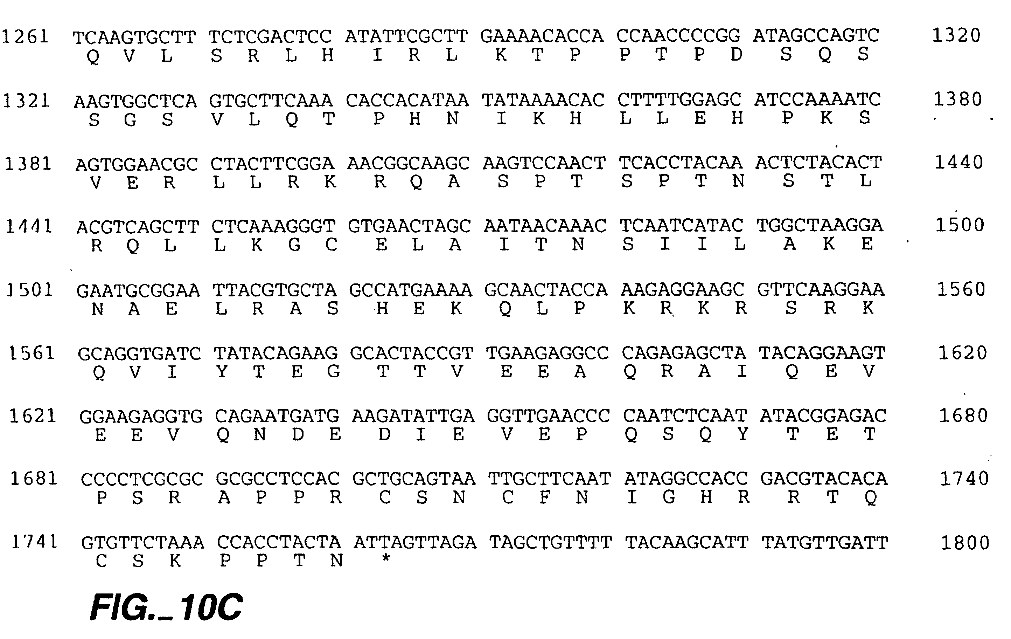

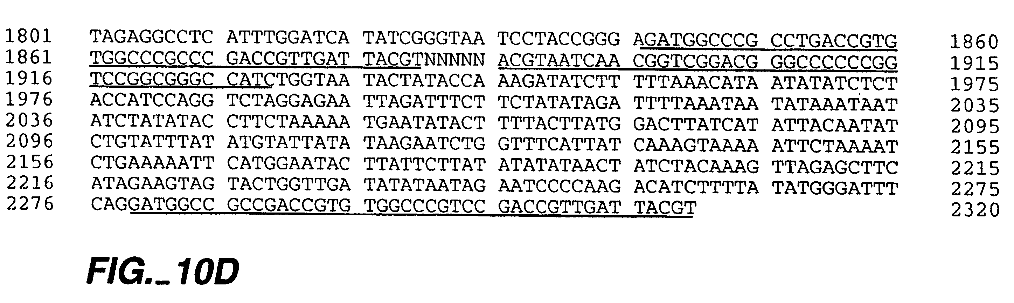

Fig. 10 shows the entire DNA sequence of the Tan element (Seq. ID No. 6), as well as the putative amino acid sequence of the transposase coded for by Tan (Seq. ID No. 7). Tan is 2320 bp in length and has a large open reading frame of 1668 bp which encodes for 555 amino adds (Seq. ID No. 7). Tan comprises the sequences of four inverted repeats (underlined) similar to those found in Vader.

### DETAILED DESCRIPTION OF THE INVENTION

While the specification concludes with claims particularly pointing out and distinctly claiming the subject matter regarded as forming the present invention, it is believed that the invention will be better understood from the following detailed description of preferred embodiments.

Standard biochemical nomenclature is used herein in which the nucleotide bases are designated as adenine (A); thymine (T); guanine (G) ; and cytosine (C). N connotes any of these nucleotides. As is conventional for convenience in the structural representation of a DNA nucleotide sequence, only one strand is usually shown in which A on one strand connotes T on its complement and G connotes C.

### EXPERIMENTAL

Applicants have demonstrated that mobile transposable elements exist in *A niger* var. *awamori*. The clone element, Vader, is present in approximately fifteen copies in the two *A. niger* strains examined. In contrast, the Vader element is present in one copy in only one of the two *A. nidulans* strains studied. These results explain why several groups have been unsuccessful in isolating active transposons in laboratory *A. nidulans* strains. A plausible assumption is that "domesticated" strains of *A. nidulans* have lost their transposons due to repeated manipulation of such strains and the possible discarding of aberrant *A. nidulans* strains displaying genetic instability. It is more difficult to explain why there are very few Vader sequences present in the other *Aspergillus* species examined.

The Vader element shows similarities to transposable elements cloned from the plant pathogens Pot1 from *M. grisea* (12) and Fot1 from *F. oxysporum (8).* The target site for duplication in all three fungi is a 2 bp TA sequence. In the case of Fot1, this transposon does not excise precisely. In two *niaD* revertants examined, the excision products retained a 4 bp insertion relative to the wild-type gene (TAATTA versus TA). The insertion studied was integrated into an intron, therefore, imprecise excision of Fot1 did not effect the functionality of the *niaD* gene product. There is no published evidence that Pot2 is a functional element.

In an attempt to determine if there were transposons similar to those reported for *F*. *oxysporum* and *M. grisea*, synthetic oligomers were made corresponding to the inverted repeats of both Fot1 (7) and Pot2 (10). When Southern analysis of *A. niger* var. *awamori* was conducted using the Vader 44 bp inverted repeat (Seq. ID No. 5) as a control, no conclusive hybridizations could be detected with either the Fot1 or Pot2 oligomeric probe. These results indicate that the Vader element is not closely related to either the previously described Fot1 and Pot2 transposons (see Fig. 7).

With regard to the structure of the Vader element, elements which transpose directly through DNA copies are typified by having inverted terminal repeats. Elements which transpose through reinsertion of the product of reverse transcription of an RNA copy of the element (retroelements) without long terminal repeats such as the *Drosphilia I* element (for a review see (16)). Alternatively, retrotransposons can have long terminal repeats such as the *Drosphilia copia* element. Vader has a 44 bp inverted terminal repeat, this indicates that Vader probably transposes through DNA copies. The Vader inverted repeats shown in Fig. 9, Seq. ID Nos. 4 and 5, respectively, have a single mismatch. Elements which transpose through DNA copies typically have open reading frame(s) which encode a transposase activity. The Fot1 element is 1.9 kb in length and the Pot1 element 1.8 kb in length. Both the Fot1 and Pot1 elements have ORF encoding for a putative transposase-like protein. The Vader element, although mobile, is too small (about 440 bp) to encode for an transposase protein. Defective elements that lack transposase activity can transpose in the presence of a transposase encoded by a complete element elsewhere in the genome.

For example, the maize Ac transposon encodes a fully functional transposase. However, maize Ds elements, although capable of excision, cannot excise unless a functional Ac element is present (17). These results indicate that for the Vader element to transpose, a transposase activity must be present in the genome of a larger transposon. Recent results using the Vader 44 bp inverted repeat (Seq. ID No. 5) to perform PCR using *A. niger* var. *awamori* genomic DNA resulted in the generation of two amplified DNA sequences, one sequence 1.8 kb in length and the second sequence of about 2.2 kb in length. This second sequence, called Tan (Seq. ID No. 6), comprises four inverted repeats similar to those in Vader. Tan is 2322 bp in length and has a large open reading frame (1668 bp) which encodes for a putative transposase comprising 555 amino acids (shown in Seq. ID No. 7).

### MATERIAL AND METHODS

Strains. Spontaneous chlorate resistant mutants were derived from *Aspergillus niger* var. *awamori* UVK143, Northern Regional Research Laboratories (NRRL# 3112). The following *Aspergillus* strains were obtained from the ATCC: *A. cinnamomoneus* (ATCC# 1027), *A. wentii* (ATCC# 10593), and A. *phoenicis* (ATCC# 11362). *A. nidulans* (FGSC# A237), a nitrate reductase structural gene mutant (*niaD*15), and *A. nidulans* (FGSC# A691), a tryptophan requiring mutant (*trpC*801), were obtained from Fungal Genetics Stock Center (FGSC). *A. versicolor, A. foetidus*, and a proprietary *A. niger glucoamylase* strain are from the Genencor International Inc. culture collection. *Escherichia coli* strains JM101 (15) and MM294 (16) were used for amplification of plasmids as described (12).

Mutant Selection. Spore suspensions (1 x 10⁸) of UVK143 were plated on CM agar (11) containing 600 mM KClO₃ and 10 mM glutamic acid. Chlorate (KClO₃), a toxic analog of nitrate, allows selection of mutants in the nitrate assimilation pathway by chlorate resistance. The small amount of glutamic acid in the medium reduces space competition between mutant and wild-type spores by inducing the nitrate assimilation pathway, and thus the uptake of the toxic chemical (chlorate) by wild-type spores. Plates were incubated at 37°C until individual colonies of spontaneous mutants could be identified. Single mutants resistant to KClO₃ were allowed to sporulate on CM plates and spores from these plates were then streaked onto minimal media (11) with various sole nitrogen sources (10 mM): NaNO₃ (nitrate), NaNO₂ (nitrite), hypoxanthine, uric acid or NH₄Cl (ammonium chloride). Each of these compounds are intermediate products of the nitrate assimilation pathway. *niaD* mutants were identified as those resistant to KClO₃ and able to grow in the presence of all pathway intermediates, except for NaNO₃.

**PCR Amplification.** Genomic DNA of *A. niger* var. *awamori niaD* mutants and UVK143 was used as template (see Southern Analysis). Primers (50 pmol) used for amplification of the *niaD* gene were NiaD1 (position 142-165 relative to the initiation site of *niaD*): *5'*-CCAACCGAGTCCTCAGTATAGAC-3' (Seq. ID No. 8) and NiaD2 (position 2738-2715): 5'-CAACGCTTCATAGGCGTCCAGATC-3' (Seq. ID No. 9). Deep Vent (exo-) DNA polymerase (New England Biolabs) was used with the buffer and dNTPs provided by the manufacturer. For optimal amplification of the *niaD* gene the reaction mixture contained 4 mM MgSO₄. Denaturation of template DNA, 2 min. at 94°C, was followed by 30 cydes of denaturation (30 sec. at 94°C), annealing of primers (45 sec. at 55°C) and extension (4 min. at 72°C). PCR fragments were purified from gel using the Qiaex DNA gel extraction kit (Qiagen), digested and used for restriction enzyme analysis by standard procedures (12).

To determine if other sequences exist which recognize the inverted repeats of Vader, a single primer (IR1) was synthesized for use with *A. niger* genomic DNA. The sequence of IR1 is 5'-ATA-TGA-ATT-CAC-GTA-ATC-AAC-GGT-CGG-ACG-GGC-CAC-ACG-GTC-AGG-CGG-GCC-ATC-3' (Seq. ID No. 10). Reaction mix included: Vader mutants and UVK143 fungal genomic DNA, Vent (exo⁻) DNA polymerase buffer, 100 pmol IR1 primer, 250 mM each of dATP, dCTP, dTTP and dGTP, DMSO, and 4 units Taq DNA polymerase. The amplifications were conducted as follows: 1 cycle of 10 min. at 94°C, 30 cycles of 1 min. at 94°C, 1 min. at 55°C, 1 min. at 72°C and one cycle of 15 min. at 72°C. Reactions were pooled, run and extracted as in the *niaD* reactions.

Template DNA from *niaD*436, derived from a purified colony grown in the presence of KClO₃, was used in a PCR reaction in an attempt to amplify both the larger *niaD* sequence with an insert and the shorter *niaD* fragment resulting from excision of the Vader element. The PCR reaction was conducted as previously described, except for using primers MA003 (positions 359-378): 5'-ATATGAATTCCTTCTTGACTTCCCCGGAAC-3' (Seq. ID No. 11) and NiaD5 (position 1125-1144): 5'-ATATAAGCTTGTCACTGGACGACATTTCAG-3' (Seq. ID No. 12). The gel purified fragment (ca. 800 bp) resulting from the excision event was submitted for sequencing.

**Estimation of *niaD* Mutant Reversion Frequency.** Spores from *niaD* mutants *niaD*392, *niaD*410, *niaD*436 and *niaD*587 were streaked onto minimal media containing NaNO₃ as a sole nitrogen source. Nitrate non-utilizing colonies of *niaD* mutants, which had a spidery appearance and did not sporulate, were streaked onto CM containing 600 mM potassium chlorate (KClO₃) and incubated to confluency at 37°C. Ten-fold dilution series of spore suspensions (in 0.8% NaCl-0.25% Tween 80) of *niaD*392, *niaD*410, *niaD*436, *niaD*587 and UVK143 wild-type spores were plated on minimal media with nitrate (10 mM) to determine reversion frequency, and on CM to determine viability.

**Southern Analysis.** Genomic DNA for PCR and Southern analysis was isolated (13) from mycelia grown in CSL (13), which contained 600 mM KClO₃ in order to reduce reversion of *niaD* back to the wild-type during cultivation. DNA (10 µg) was digested with either Bglll, which leaves the insertion intact in the *niaD* gene, or with EcoRV, which cuts the insertion element (Vader) once, and thus enables determination of its copy number in the genome. Genomic DNA (approximately 10 µg) of *A. nidulans, A. cinnamomeus*, *A. versicolor, A. wentii, A. phoenicis, A. foetidus* and of an industrial *A. niger* strain were digested with EcoRV to obtain an estimate of Vader copy number in these fungal genomes. The digested and gel-separated DNA was transferred to a positively-charged nylon membrane (Boehringer Mannheim) by capillary action.

The DNA probe for the *niaD* gene was derived from the PCR product (UVK143 DNA template amplified with primers NiaD1 (Seq. ID No. 8) and NiaD2 (Seq. ID No. 9)), which was digested with Sall, resulting in a 528 bp probe fragment. The probe for the insertion element, Vader, was derived from a PCR reaction in which *niaD*436 DNA was used as a template. This PCR product was purified and digested with Sall and Sphl and subcloned into the vector pUC19. This subclone was digested with Scal and Xbal to yield a 236 bp fragment which was used for estimation of the copy number of Vader sequences in the genomes of various fungi.

A DNA labeling and detection kit (Genius1, Boehringer Mannheim) was used for random primed labeling of probe DNA with digoxigenin, and for detection with alkaline-phosphatase labeled antibody to digoxigenin.

Hybridization and washing conditions for homologous probes were conducted as recommended by the manufacturer using hybridization buffer without formamide at 68°C (Boehringer Mannheim). Hybridizations for heterologous Southern analysis (i.e., analysis of DNA from other *Aspergillus* sp.) was conducted using hybridization buffer with 25% formamide at 37°C. Washes were performed as in stringent wash protocol.

**Nitrate Reductase Assays.** Nitrate reductase assays were performed as described in Dunn-Coleman, et al. (18).

**DNA analysis and Sequence Determination.** Sequences were determined using dye Deoxy terminators and *Taq* cycle sequencing on the 373A sequencer (ABI). Commercially available universal and reverse (New England Bioiabs) primers were used. Alignment of sequences and prediction of amino acid sequences were performed using DNASTAR (DNASTAR, Inc.). The nucleotide and deduced amino acid sequences were analyzed and compared to those in GenBank, EMBL and Prot-Swiss using the Genetics Computer Group, Inc. software package (Madison, Wl).

### Example 1

### Isolation of Spontaneous High Frequency Reverting niaD Mutants of A. niger var. awamori

Assuming that *niaD* mutants which arise from the insertion of a transposable element would be unstable, a total of 152 *niaD* mutants, isolated on the basis of spontaneous resistance to chlorate were characterized. To determine if the *niaD* mutation was unstable, spores from 43 *niaD* mutants were plated onto medium with nitrate as the sole nitrogen source. Fourteen of the mutants reverted to the wild-type phenotype at a frequency of greater than 1 X 10⁵. Table 1 summarizes the *niaD* mutant reversion studies.

**Table 1**

| Mutant | Conidiaplated No. x 10³ | No. Wild-Type Colonies | Reversion Frequency x 10⁻⁴ |
|---|---|---|---|
| *niaD*392 | 2.9 | 27 | 93 |
| *niaD*410 | 7.7 | 5 | 6.5 |
| *niaD*436 | *3.7* | 164 | 443 |
| *niaD*587 | 18.9 | 12 | 6.3 |

There appeared to be two classes of *niaD* mutants which reverted at high frequency. The *niaD* mutants *niaD*436 and *niaD*392 reverted at high frequency, while mutants *niaD*410 and *niaD*587 yielded smaller numbers of revertant colonies.

The level of nitrate reductase activity was determined by revertant colonies isolated from two *niaD* mutants. Nitrate reductase activity was detected in all six *niaD*410 mutants In the case of *niaD*436 revertants, nitrate reductase activity could be detected in 3 of 6 revertants analyzed.

### Example 2

### Cloning of a Vader Element

To determine if an insertion sequence was located within the *niaD* gene, two primers were synthesized. The first primer, niaD1 (Seq. ID No. 8), corresponded to position 142-165 of the *niaD* gene, and niaD2 (Seq. ID No. 9) corresponded to position 2738-2715 of the *niaD* gene. Genomic DNA was isolated from 14 unstable *niaD* mutants. This genomic DNA served as a template for the PCR primers. PCR reaction products with 4 *niaD* mutants (410, 436, 587 and 392) revealed an approximately 440 bp insertion (Vader) in the *niaD* gene.

For Southern blot analysis, genomic DNA isolated from the wild-type and four *niaD* mutants (410, 436, 587 and 392) was digested with BglII. The probe used was a Sal1 digestion fragment of the 500 bp PCR product generated using the niaD1 (Seq. ID No. 8) and niaD2 (Seq. ID No. 9) oligomeric probes (see Fig. 1). The probe hybridized to a 2.5 kb fragment with wild-type DNA (lane 5). In the case of the *niaD* mutants 410 (lane 1), 436(lane 3) and 392 (lane 4), the probe hybridized to a 2.9 kb fragment. These results indicate that these three *niaD* mutants contain an approximately 440 bp insertion. Interestingly, with the mutant *niaD*587, the probe hybridized to both a 2.5 kb and 2.9 kb fragment. Although, mycelium had been grown in the experiment in the presence of KClO₃ to favor growth of the *niaD* mutant and not reverent cells, the detection of two hybridizable sequences indicated that in some cells Vader had been excised from the *niaD* gene.

The approximate location of the insertion was determined in each of the four unstable *niaD* mutants by restriction mapping analysis. The location of the insertion in each of the four mutants examined is shown in Fig. 2. All four mutants had an approximately 440 bp insertion located at different sites within the *niaD* gene.

### Example 3

### Determination of Vader Copy Number

To determine the Vader copy number a 236 bp Sca1-Xba1 internal fragment of Vader-2 (cloned from the mutant *niaD*436) was hybridized to EcoRV cleaved genomic DNA. There is only one EcoRV site within the Vader transposon (Fig. 2). Southern blot analysis indicated that there are approximately fifteen copies of Vader sequences in the genome of *A. niger var. awamori*. (Fig. 4). The Vader sequences were integrated at identical genomic locations in the three *niaD* mutants, 410, 436 and 587. However, in the *niaD*392 mutant, Vader sequences were located in five different locations compared to the three *niaD* mutants examined. This result was somewhat surprising considering that all four *niaD* mutants were isolated from the same strain, but provides good evidence for the high mobility of the Vader element in this strain. When a propriety *A. niger* glucoamylase production strain (ETC# 2663) was also examined, approximately 15 hybridization signals could be detected. Although some of the hybridization patterns appeared to be identical, clear differences could be seen between *A. niger* var. *awamori* and *A. niger.*

### Example 4

### Isolation of Vader in Other Fungal Species

In an attempt to determine if this transposable element was found in other filamentous fungi, genomic Southern blot analysis was performed using the 236 bp fragment (Xbal-Scal) of Vader sequence as per Example 3, as a probe (Fig. 5). Two strains of *A. nidulans* were obtained from Fungal Genetics Stock Center (FGSC), FGSC# A691, a nitrate reductase structural gene mutant (*niaD*15), and FGSC# A237, a tryptophan-requiring mutant (*trp*C801). No hybridization signals could be visualized with strain A691, and a single strong hybridization signal could be detected with strain A691. These results support the notion that the lack of success in cloning transposable elements from laboratory strains of *A. nidulans* is due to low copy number or absence. Similarly, only 2 hybridization signals could be detected in *A. foetidus* and *A. phoenicis,* and 1 hybridization signal was detected in *A. cinnamomeus*. No hybridizations could be detected in *A. wentii* and *A. versicolor*. In addition, no hybridization signals could be detected with *Humicola grisea* var. *thermoidea*, *Neurospora crassa* and *Trichoderma reesei* (results not shown). These results indicate that the Vader element is most commonly found in *A. niger* var. *awamori* and *A. niger.*

### Example 5

### Excision of the Vader Element

Part of the *niaD* gene from *niaD*436 containing the Vader element was amplified using PCR. The PCR amplification resulted in the expected 1200 bp fragment of the Vader element flanked by *niaD* sequences and a shorter 800 bp fragment resulting from the excision event. Sequencing of the shorter fragment indicated that the Vader element had excised precisely. However, when several revertants of *niaD*436 and *niaD*410 were assayed for their nitrate reductase activity (18), a spectrum of activities were detected (results not shown).

### Example 6

### Insertional Inactivation/Gene Tagging

Vader was cloned by insertional inactivation of the target gene *niaD,* which encodes nitrate reductase. The target sequence for integration of Vader is TA, a sequence which must be very common in the genome of fungi. Nitrate reductase mutants cannot grow on nitrate and inconsequence are resistant to the toxic analog of nitrate, KClO₃.

It is possible that one of the reasons heterologous protein production in fungi is lower than that of homologously produced protein using the same promoter is that the heterologous protein is being degraded by the cell. If there are genes whose products are responsible for degrading/sequestering foreign protein, it would be advantageous to inactivate those genes. In order to achieve this, a strain is constructed using gene disruption, which lacks the Tan gene. Such strain is then used to transform and express a heterologous protein such as the mammalian chymosin protein. It would be advantageous if the activity of such genes could be visualized or selected for on petri dishes. For example chymosin produced in *A. niger* results in a halo of clearing around a colony grown on skim milk. (See US Patent 5,364,770, the disclosure of which is incorporated herein by reference.) Having transformed the strain with a construct comprising the desired heterologous protein or polypeptide, one would transform the strain a second time with the transposon Tan located on the replication vector pHELP (20) (as per Scheme 1 below). Once the strain has been transformed a second time, a spore suspension is made from transformants. Genencor International, Inc. has shown that the pHELP plasmid is not found in spores (i.e., when spores are made, the pHELP plasmid is excluded from the spore) (unpublished). Making a spore suspension will then "freeze" the transposon in the genome, i.e., it will not be able to move again after the spore isolation phase because the transposase required for excision is now missing from the cell.

The transformants are then plated on medium which can be used to visualize heterologous protein production, such as skim milk plates in the case of chymosin.

The plates are then screened for increased halo size, which is the result of inactivation of a gene whose product limits foreign protein production.

The inactivated gene can be cloned using the transposon sequences as a marker for cloning strategies. (See generally (19).)

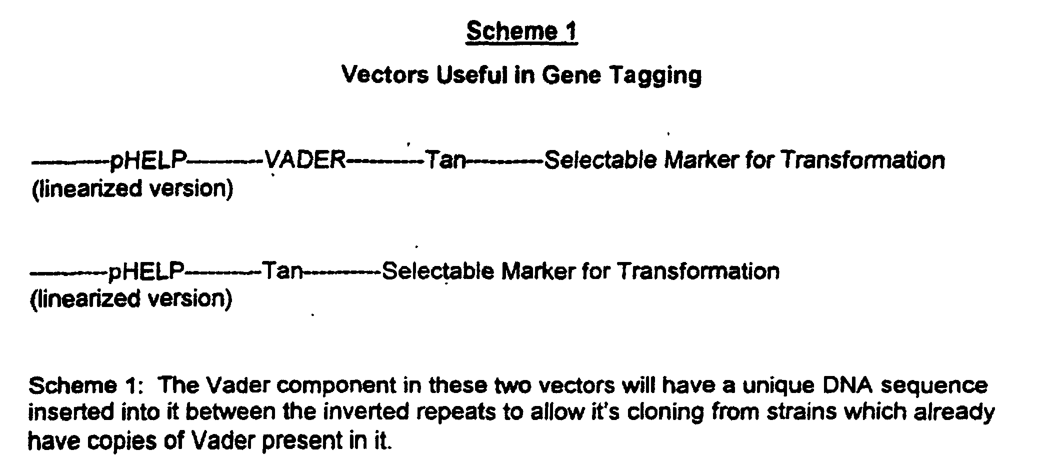

### Example 7

### Elevation of Gene Expression Using Transposons

A reason that heterologous protein production is lower than expected in fungi is presumed to be that genes essential for foreign (heterologous) gene production are NOT expressed at sufficiently high levels in the fungi.

In order to overcome this problem, utilizing the transposable element(s) of the present invention, a strain is constructed in which the native Tan gene is inactivated by gene disruption.

This strain is used to express a heterologous protein whose expression can be easily visualized, such as chymosin (US Patent 5,364,770) using the following vector:

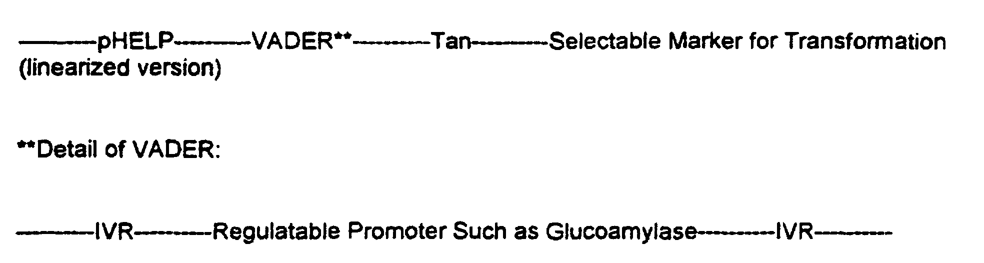

One of the many integration events possible will be the integration of the Vader element 5' to a gene, which if elevated in activity by using a very strong promoter such as glucoamylase, will enhance the secretion of the heterologous protein (i e., chymosin) For example, see Scheme 2 below.

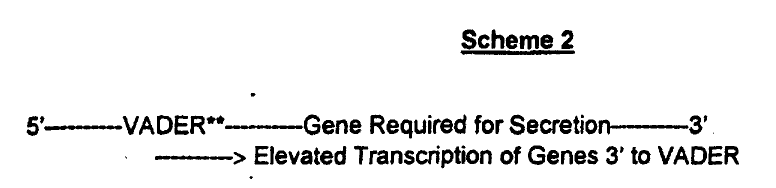

Spore suspensions will be isolated from transformants. As previously discussed, the spore isolation step results in the elimination of the pHELP (20) vector from the strain and "freezes" the transposon integrated into the genome. The spores are then plated out and transformants which make more chymosin characterized. Then, using conventional cloning strategies, the gene 3' to Vader can be doned. (See generally (19), incorporated herein by reference.)

### References.

1. Schectman, M.G. (1987) *Mol. Cell. Biol*. **7:**3168-3177
2. Kinsey, J.A. & Hebler, J. (1989) *Proc. Natl. Acad. Sci. USA* **86:**1929-1933
3. Kinsey, J.A. (1993) *Proc. Natl. Acad. Sci. USA* **90:**9384-9387
4. Cambareri, E.B., Helber, J. & Kinsey, J.A. (1994) *Mol. Gen. Genet.* **242**:658-665
5. McHale, M.T., Roberts, I.N., Noble, S.M., Beaumont, C., Whitehead, M.P., Seth, D. & Oliver, R.P. (1992) *Mol. Gen. Genet.* **233**:337-347
6. Daboussi, M.J., Langin, T. & Brygoo, Y. (1992) *Mol. Gen. Genet.* **232**:12-16
7. Cove, D.J. (1976) Heredity **36**:191-203
8. Daboussi, M.J. & Langin, T. (1994) *Genetica* **93:**49-59
9. Lebrum, M.-H. Chumley, F. & Valent, B. (1994) *Fungal Genetics News Letter* **41A**:52
10. Kachroo, P., Leong, S.A. & Chattoo, B.B. (1994) *Mol. Gen. Genet.* **245**:339-348
11. Rowlands, R.T. & Turner, G. (1973) *Mol. Gen. Genet.* **126**:201-216
12. Sambrook, J., Fritsch, E.F. & Maniatis, T. (1989) Molecular Cloning: A Laboratory Manual, Cold Spring Harbor Laboratory Press, Plainview, NY, pp. 1.11-1.85
13. Timberlake, W.E. & Bamard, E.C. (1981) *Cell* **26**:29-37.
14. Yanisch-Peron, C., Vieira, J. & Messing, J. (1985) *Gene* **33**:103-119
15. Meseselson, M. & Yuan, R. (1968) *Nature* **217:**1110-1114
16. Charteswarth, B., Snlegowski, P. & Stephan, W. (1994) *Nature* **371**:215-220.
17. Fedoroff, N,V., Furtek. D.B. & Nelson, O.E. (1984) *Proc. Natl Acad*. *Sci. USA* **81**:3829-3835
18. Dunn-Coleman, N.S., Tomsch, A.D. & Garrett, R.H. (1981) *Molec. Gen. Getet.* **182**:234-239
19. Walden, R. & Schell, J. (1994) *Agro-Food-Industry-Hi-Tech,* **Nov/Dec**:9-12
20. Gems, D.H., Johnstone, I.L. & Clutterbuck, A.J. (1991) *Gene* **98**:61-67

## Claims

1. A transposable element comprising a repeat sequence as set out in SEQ ID NO: 4, SEQ ID NO: 5, or any one of the underlined sequences in Figure 10.

2. A transposable element according to claim I comprising a repeat of each of SEQ ID NO: 4 and SEQ ID NO: 5.

3. A transposable element according to claim 1 or claim 2, wherein the transposable element is 440bp in length.

4. A transposable element according to claim 3 having the sequence of SEQ ID NO: 3.

5. A transposable element according to claim 1 wherein the transposable element is 2.2kb or 2.4kb in length.

6. A transposable element according to claim 5, having the sequence of SEQ ID NO: 6.

7. A transposase encoded by a transposable element according to claim 5 or claim 6.

8. A transposase according to claim 7 comprising the amino acid sequence of SEQ ID NO: 7.

9. A method of isolating a transposable element from a filamentous fungus, comprising the steps of hybridising a probe, comprising the sequence of SEQ ID NO: 4, SEQ ID NO: 5 or any one of the underlined sequences in Figure 10, to fungal DNA, and isolating DNA which hybridises to the probe.

10. A method of isolating a transposable element from a filamentous fungal genomic library, comprising the steps of probing said library with a probe corresponding to the Tan open reading having the nucleic acid sequence set out in SEQ ID NO 6.

11. A method of isolating a transposable element from a filamentous fungus, comprising the steps of using a DNA fragment having the sequence of SEQ ID NO: 4 or SEQ ID NO: 5 as a primer in a polymerase chain reaction amplification of fungal DNA, generating amplified DNA sequences and optionally characterising said amplified DNA.

12. A method of inactivating a gene, comprising the steps of transforming a transposable element according to any one of claims 1 to 6 into a cell containing said gene, wherein said element becomes inserted into said gene, disrupting expression from said gene.

13. A method of activating a gene, comprising the steps of transforming a transposable element according to any one of claims 1 to 6 into a cell containing said gene, the transposable element having inserted therein a DNA sequence capable of regulating expression of said gene, wherein said transposable element becomes inserted into the genome of the cell such as to regulate the expression of said gene.

## Patentansprüche

1. Transponierbares Element, umfassend eine repetitive Sequenz wie in Seq.-ID Nr. 4, Seq.-ID Nr. 5 oder einer der unterstrichenen Sequenzen in Fig. 10 dargelegt.

2. Transponierbares Element nach Anspruch 1, das eine Wiederholung einer jeden von Seq.-ID Nr. 4 und Seq.-ID Nr. 5 umfasst.

3. Transponierbares Element nach Anspruch 1 oder 2, worin das transponierbare Element eine Länge von 440 bp aufweist.

4. Transponierbares Element nach Anspruch 3, das die Sequenz von Seq.-ID Nr. 3 aufweist.

5. Transponierbares Element nach Anspruch 1, worin das transponierbare Element eine Länge von 2,2 kb oder 2,4 kb aufweist.

6. Transponierbares Element nach Anspruch 5, das die Sequenz von Seq.-ID Nr. 6 aufweist.

7. Transposase, für die ein transponierbares Element nach Anspruch 5 oder 6 kodiert.

8. Transposase nach Anspruch 7, welche die Aminosäuresequenz von Seq.-ID Nr. 7 umfasst.

9. Verfahren zum Isolieren eines transponierbaren Elements aus einem Fadenpilz, umfassend die Schritte des Hybridisierens einer Sonde, die die Sequenz von Seq.-ID Nr. 4, Seq.-ID Nr. 5 oder einer der unterstrichenen Sequenzen in Fig. 10 umfasst, an Pilz-DNA, und das Isolieren von DNA, die an die Sonde hybridisiert.

10. Verfahren zum Isolieren eines transponierbaren Elements von einer Fadenpilz-Genombank, umfassend die Schritte des Sondierens der Bank mit einer Sonde, die dem offenen Tan-Leseraster mit der in Seq.-ID Nr. 6 dargelegten Nucleinsäuresequenz entspricht.

11. Verfahren zum Isolieren eines transponierbaren Elements aus einem Fadenpilz, umfassend die Schritte der Verwendung eines DNA-Fragments mit der Sequenz von Seq.-ID Nr. 4 oder Seq.-ID Nr. 5 als Primer in einer Polymerase-Kettenreaktions-Amplifizierung von Pilz-DNA, des Erzeugens amplifizierter DNA-Sequenzen und gegebenenfalls des Charakterisierens der amplifizierten DNA.

12. Verfahren zum Inaktivieren eines Gens, umfassend die Schritte des Transformierens einer transponierbaren Elements nach einem der Ansprüche 1 bis 6 in eine Zelle, die das Gen enthält, wobei das Element in das Gen insertiert wird, wodurch die Expression des Gens unterbrochen wird.

13. Verfahren zum Aktivieren eines Gens, umfassend die Schritte des Transformierens eines transponierbaren Elements nach einem der Ansprüche 1 bis 6 in eine Zelle, die das Gen enthält, wobei im transponierbaren Element eine DNA-Sequenz insertiert ist, die zum Regulieren der Expression des Gens fähig ist, worin das transponierbare Element in das Genom der Zelle insertiert wird, um die Expression des Gens zu regulieren.

## Revendications

1. Elément transposable comprenant une séquence de répétition telle qu'indiquée dans SEQ ID NO:4, SEQ ID NO:5, ou n'importe laquelle des séquences soulignées de la Figure 10.

2. Elément transposable selon la revendication 1, comprenant une répétition de chacune de SEQ ID NO:4 et SEQ ID NO:5.

3. Elément transposable selon la revendication 1 ou la revendication 2, où l'élément transposable a 440 pb de long.

4. Elément transposable selon la revendication 3, ayant la séquence de SEQ ID NO:3.

5. Elément transposable selon la revendication 1, où l'élément transposable a 2,2 kb ou 2,4 kb de long.

6. Elément transposable selon la revendication 5, ayant la séquence de SEQ ID NO:6.

7. Transposase codée par un élément transposable selon la revendication 5 ou la revendication 6.

8. Transposase selon la revendication 7, comprenant la séquence d'acides aminés de SEQ ID NO:7.

9. Méthode d'isolement d'un élément transposable à partir d'un champignon filamentaire, comprenant les étapes d'hybrider une sonde, comprenant la séquence de SEQ ID NO:4, SEQ ID NO:5 ou n'importe laquelle des séquences soulignées de la Figure 10, à de l'ADN fongique et d'isoler l'ADN qui s'hybride à la sonde.

10. Méthode pour isoler un élément transposable à partir d'une bibliothèque génomique fongique filamentaire, comprenant les étapes de sonder ladite bibliothèque avec une sonde correspondant au cadre de lecture ouvert de Tan ayant la séquence d'acide nucléique indiquée à SEQ ID NO: 6.

11. Méthode d'isolement d'un élément transposable d'un champignon filamentaire, comprenant les étapes d'utiliser un fragment d'ADN ayant la séquence de SEQ ID NO:4 ou SEQ ID NO:5 en tant qu'amorce dans une amplification par réaction de chaîne de polymérase de l'ADN fongique, générer des séquences d'ADN amplifié et caractériser facultativement ledit ADN amplifié.

12. Méthode pour inactiver un gène, comprenant les étapes de transformer un élément transposable selon l'une quelconque des revendications 1 à 6 en une cellule contenant ledit gène, où ledit élément se trouve inséré dans ledit gène, rompant l'expression dudit gène.

13. Méthode pour activer un gène, comprenant les étapes de transformer un élément transposable selon l'une quelconque des revendications 1 à 6 en une cellule contenant ledit gène, dans l'élément transposable étant insérée une séquence d'ADN capable de réguler l'expression dudit gène, où ledit élément transposable se trouve inséré dans le génome de la cellule de façon à réguler l'expression dudit gène
